# EUROPEAN PATENT APPLICATION

(11) **EP 4 428 533 A1**
(43) Date of publication of application: **11.09.2024**
(21) Application number: 22889957.1
(22) Date of filing: 01.11.2022
(51) Int. Cl.: G01N 33/536, C12N 15/11, C12Q 1/6813

(54) **METHOD FOR DETECTING TARGET SUBSTANCE**

(30) Priority: 04.11.2021 JP 2021180020
(71) Applicant: Toppan Holdings Inc., Tokyo 110-0016 (JP)
(72) Inventor: KUBO, Yuji, Tokyo 110-0016 (JP); MAKINO, Yoichi, Tokyo 110-0016 (JP)
(74) Representative: Plasseraud IP
(86) International application number: PCT/JP2022/040892
(87) International publication number: WO 2023/080137

(57) **Abstract**

A method of detecting a target substance (110) in a sample includes a step of introducing into wells the sample, a first specific binding substance (120) which is for the target substance (110) and labeled with a first single-stranded nucleic acid fragment (121), and a second specific binding substance (130) which is for the target substance (110) and labeled with a second single-stranded nucleic acid fragment (131), forming as a result a complex (100) containing the target substance (110), the first specific binding substance (120), and the second specific binding substance (130) if the target substance (110) is present in the sample, and forming a double-stranded nucleic acid (140) by hybridization of at least part of the first single-stranded nucleic acid fragment (121) with at least part of the second single-stranded nucleic acid fragment (131); and a step of detecting formation of the double-stranded nucleic acid (140), wherein detection of formation of the double-stranded nucleic acid (140) indicates presence of the target substance (110).

## Description

### [Technical Field]

The present invention relates to methods of detecting a target substance. More specifically, the present invention relates to methods of detecting a target substance, and kits for detecting a complex and a target substance.

The present application claims the benefit of priority from Japanese Patent Application No. 2021-180020 filed November 4, 2021, the content of which is incorporated herein by reference.

### [Background Art]

Quantitative detection of a target substance in a biological sample enables early detection of diseases and prediction of medication efficacy. Conventionally, protein quantification has been performed using enzyme-linked immunosorbent assays (ELISAs), etc., and nucleic acid quantification has been performed using real-time PCR methods etc.

In recent years, there is an increasing need for detecting target substances with higher accuracy for the purpose of detecting diseases at an earlier stage or for other purposes. As a method of detecting a target substance with higher accuracy, techniques for causing enzymatic reactions in a large number of microcompartments are being considered. These techniques are called digital measurements. Digital measurements include digital ELISAs and digital PCRs.

In digital measurements, a sample solution is divided into an extremely large number of micro-solutions. Then, signals from the individual micro-solutions are binarized and only whether the target substance is present is determined to measure the number of molecules of the target substance. Digital measurements can significantly improve detection sensitivity and quantifiability compared to ELISAs, real-time PCRs, etc. of conventional art.

For example, Non-Patent Literature (NPTL) 1 describes a microwell array with microwells and channels for supplying reagents, etc., and describes performing a digital ELISA using the microwell array.

PTL 1 and NPTLs 2 and 3 describe a proximity ligation assay (PLA) and proximity extension assay (PEA) which are methods of detecting proteins using antibodies modified with oligonucleotides. These methods utilize PCR methods for the detections.

### [Citation List]

### [Patent Literature]

PTL 1: WO2012/160083

### [Non-Patent Literature]

NPTL 1: Kan C. W., et al., Isolation and detection of single molecules on paramagnetic beads using sequential fluid flows in microfabricated polymer array assemblies, Lab on a Chip, 12 (5), 977-985, 2012.
NPTL 2: Mohammed H., et al., Approaches for Assessing and Discovering Protein Interactions in Cancer., Mol Cancer Res, 11 (11), 1295-1302, 2013.
NPTL 3: Assarsson E., et al., Homogenous 96-Plex PEA Immunoassay Exhibiting High Sensitivity, Specificity, and Excellent Scalability, PLoS One, 9 (4), e95192, 2014.

### [Summary of the Invention]

### [Technical Problem]

ELISAs require a process of washing unreacted molecules of antibody after reaction of the target substance with the antibody. However, in devices used for digital measurements, it is difficult to perform the washing process due to the target substance or the antibody being sealed in the wells or droplets.

NPTL 1 describes that a PSA, i.e., a detection target, is bound to magnetic beads, sequentially followed by washing the beads, reacting the beads with a biotin-labeled antibody, washing the beads, reacting the beads with a solution containing streptavidin-P-galactosidase, washing the beads, suspending the beads in a buffer containing a substrate, and introducing the beads into the well array to detect the target. Thus, detecting a target substance using a digital ELISA may involve complicated pre-processing. The present invention aims to provide a technique for detecting a target substance without performing a washing process.

### [Solution to Problem]

Aspects of the present invention are as follows.
[1] a method of detecting a target substance in a sample, including a step of introducing into wells the sample, a first specific binding substance which is for the target substance and labeled with a first single-stranded nucleic acid fragment, and a second specific binding substance which is for the target substance and labeled with a second single-stranded nucleic acid fragment, forming as a result a complex containing the target substance, the first specific binding substance, and the second specific binding substance if the target substance is present in the sample, and forming a double-stranded nucleic acid by hybridization of at least part of the first single-stranded nucleic acid fragment with at least part of the second single-stranded nucleic acid fragment; and a step of detecting formation of the double-stranded nucleic acid, wherein detection of formation of the double-stranded nucleic acid indicates presence of the target substance.
[2] The method according to [1], wherein the wells each have a volume of 10 fL to 100 pL.
[3] The method according to [1] or [2], wherein the first specific binding substance recognizes a first binding site of the target substance and the second specific binding substance recognizes a second binding site of the target substance, and the first binding site and the second binding site are different from each other.
[4] The method according to [3], wherein a distance between the first binding site and the second binding site is a distance at which at least part of the first single-stranded nucleic acid fragment can be hybridized with at least part of the second single-stranded nucleic acid fragment.
[5] The method according to any of [1] to [4], wherein the first single-stranded nucleic acid fragment and the second single-stranded nucleic acid fragment each have a base length of 10 to 200 bases.
[6] The method according to any of [1] to [5], further comprising a step of sealing openings of the wells after introducing the sample, the first specific binding substance, and the second specific binding substance into the wells.
[7] The method according to [6], wherein the wells whose openings are sealed include molecules of the first specific binding substance unbound to the target substance and/or molecules of the second specific binding substance unbound to the target substance.
[8] The method according to any of [1] to [7], wherein the target substance is a substance selected from a group consisting of proteins, sugar chains, nucleic acids, lipid membrane structures, bacteria, viruses, and cells, or a composite substance thereof.
[9] The method according to any of [1] to [8], wherein the first specific binding substance and the second specific binding substance each are a substance selected from a group consisting of antibodies, lectins, and substances bindable to lipid membranes.
[10] The method according to any of [1] to [9], wherein the step of detecting formation of the double-stranded nucleic acid is performed through an invasive cleavage assay.
[11] A complex containing a target substance, a first specific binding substance which is for the target substance and labeled with a first single-stranded nucleic acid fragment, and a second specific binding substance which is for the target substance and labeled with a second single-stranded nucleic acid fragment, and forming a double-stranded nucleic acid by hybridization of at least part of the first single-stranded nucleic acid fragment with at least part of the second single-stranded nucleic acid fragment.
[12] A kit for detecting a target substance, including a well array including a plurality of wells; a first specific binding substance which is for the target substance and labeled with a first single-stranded nucleic acid fragment; and a second specific binding substance which is for the target substance and labeled with a second single-stranded nucleic acid fragment.
[13] The kit according to [12], further comprising a sealing liquid for sealing the openings of the wells.
[14] The kit according to [12] or [13], further comprising a reagent for detecting a double-stranded nucleic acid formed by hybridization of at least part of the first single-stranded nucleic acid fragment with at least part of the second single-stranded nucleic acid fragment.

### [Advantageous Effects of the Invention]

According to the present invention, a technique for detecting a target substance without performing a washing process can be provided.

### [Brief Description of the Drawings]

Fig. 1 is a schematic diagram illustrating a method of detecting a target substance.
Fig. 2 is a schematic cross-sectional view illustrating an example of a fluidic device.
Fig. 3 is a schematic cross-sectional view illustrating an example of a fluidic device.
Fig. 4 is a schematic cross-sectional view illustrating an example of a fluidic device.
Fig. 5 is a schematic cross-sectional view illustrating an example of a fluidic device.
Fig. 6 is a schematic cross-sectional view illustrating an example of a fluidic device.
Fig. 7 is a schematic cross-sectional view illustrating an example of a fluidic device.
Fig. 8 is a schematic diagram illustrating an example of an invasive cleavage assay (ICA) method.
Fig. 9 is a graph showing the results of Experimental Example 1.
Fig. 10 is a graph showing the results of Experimental Example 1.
Fig. 11 is a graph showing the results of Experimental Example 1.
Fig. 12 shows microscopic images showing the results of Experimental Example 2.
Fig. 13 are graphs showing the results of Experimental Example 2.
Fig. 14 is a graph showing average well brightness ± standard deviation in the case where a target substance with each concentration is detected in Experimental Example 2.
Fig. 15 is a graph showing the number of wells indicating brightness of set value or more in the case where a target substance with each concentration is detected in Experimental Example 2.
Fig. 16 is a graph showing the results of immuno-ICA reaction using oligonucleotide-modified antibodies conjugated to DNA 1 and DNA 2 in Experimental Example 3.
Fig. 17 is a graph showing the results of immuno-ICA reaction using oligonucleotide-modified antibodies conjugated to DNA 2 and DNA 3 in Experimental Example 3.
Fig. 18 is a graph showing the results of immuno-ICA reaction using oligonucleotide-modified antibodies conjugated to DNA 2 and DNA 4 in Experimental Example 3.
Fig. 19 is a graph showing the results of immuno-ICA reaction using oligonucleotide-modified antibodies conjugated to DNA 2 and DNA 5 in Experimental Example 3.
Fig. 20 is a graph showing the results of immuno-ICA reaction using oligonucleotide-modified antibodies conjugated to DNA 2 and DNA 5 in Experimental Example 4.

### [Description of the Embodiments]

With reference to the drawings where necessary, embodiments of the present invention will be described in detail. In the drawings, the same or corresponding components are denoted by the same or corresponding reference numerals to omit repeated description. The dimension ratios in some drawings may be exaggerated for convenience of explanation and are not necessarily to scale.

### [Method of detecting target substance]

In an embodiment, the present invention provides a method of detecting a target substance in a sample, including a step of introducing into wells the sample, a first specific binding substance which is for the target substance and labeled with a first single-stranded nucleic acid fragment, and a second specific binding substance which is for the target substance and labeled with a second single-stranded nucleic acid fragment, forming as a result a complex containing the target substance, the first specific binding substance, and the second specific binding substance if the target substance is present in the sample, and forming a double-stranded nucleic acid by hybridization of at least part of the first single-stranded nucleic acid fragment with at least part of the second single-stranded nucleic acid fragment; and a step of detecting formation of the double-stranded nucleic acid, wherein detection of formation of the double-stranded nucleic acid indicates presence of the target substance.

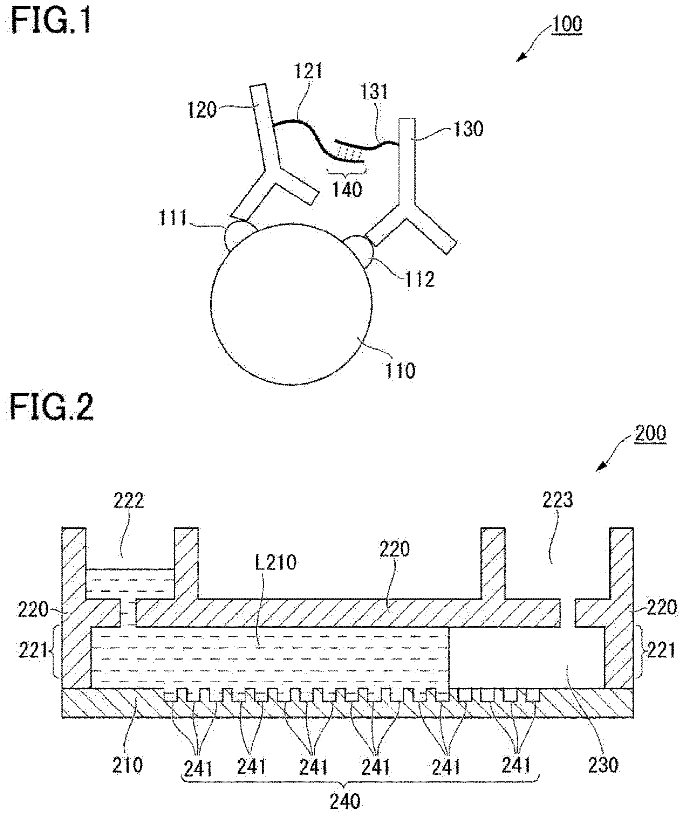

Fig. 1 is a schematic diagram illustrating a method according to the present embodiment. As shown in Fig. 1, in the method of the present embodiment, first, a sample, a first specific binding substance 120 labeled with a first single-stranded nucleic acid fragment 121, and a second specific binding substance 130 labeled with a second single-stranded nucleic acid fragment 131 are introduced into wells. Consequently, if a target substance 110 is present in the sample, a complex 100 containing the target substance 110, first specific binding substance 120, and second specific binding substance 130 is formed. At least part of the first single-stranded nucleic acid fragment 121 hybridizes with at least part of the second single-stranded nucleic acid fragment 131 to form a double-stranded nucleic acid (also referred to as a double-stranded nucleic acid region) 140.

Subsequently, formation of the double-stranded nucleic acid 140 is detected. If formation of the double-stranded nucleic acid 140 is detected, it can be said that the target substance 110 is present. The method according to the present embodiment may be applied to a sample containing no target substance 110. In this case, formation of the double-stranded nucleic acid 140, i.e., presence of the target substance 110, is not detected. Detecting formation of the double-stranded nucleic acid 140 will be described later.

As shown in Fig. 1, the first specific binding substance 120 recognizes a first binding site 111 of the target substance 110. The second specific binding substance 130 recognizes a second binding site 112 of the target substance 110. The first binding site 111 and the second binding site 112 may be different from each other. For example, if the target substance is a protein, the first binding site 111 and the second binding site 112 can also be respectively referred to as a first epitope 111 and a second epitope 112.

The sample may be a biological sample, an environmental sample, or the like. Examples of the biological sample include serum, plasma, and urine, but are not specifically limited thereto. Examples of the environmental sample include river water and factory wastewater. The target substance 110 may be a substance selected from the group consisting of proteins, sugar chains, nucleic acids, lipid membrane structures, bacteria, viruses, and cells, or a composite substance thereof.

The proteins may be glycoproteins or membrane proteins. The proteins may be composite substances. Examples of the composite substances include glycoproteins in which sugar chains are bound to proteins, and fusion proteins in which multiple proteins are fused. The lipid membrane structures may be vesicles composed of artificial or natural lipids, examples of which include liposomes, exosomes, and intracellular organelles. When detecting the lipid membrane structures, bacteria, viruses, or cells, proteins, sugar chains, nucleic acids, or the like present in these target molecules may be detected.

In the target substance 110, the distance between the first and second binding sites 111 and 112 is preferred to be a distance at which at least part of the first single-stranded nucleic acid fragment 121 can be hybridized with at least part of the second single-stranded nucleic acid fragment 131. The distance at which hybridization can be achieved is preferred to be a distance at which the first and second single-stranded nucleic acid fragments 121 and 131 can come close to each other for hybridization.

For example, assuming the target substance 110 to be a sphere with a diameter of 10 nm, the distance between the first and second binding sites 111 and 112 is preferred to be 3 nm to 10 nm, and more preferred to be 3.5 nm to 9 nm. As an example, assuming the target substance 110 to be a sphere with a diameter of 10 nm, and if the angle between the first and second binding sites 111 and 112 with reference to the center of the target substance 110 is 30° to 120°, the distance between the first and second binding sites 111 and 112 may be 4.14 nm to 8.66 nm. Herein, the distance between the first and second binding sites 111 and 112 is defined to be a minimum distance therebetween parallel to the surface of the target substance 110.

Specifically, for example, the base length of the first single-stranded nucleic acid fragment 121 may be 10 to 200 bases. Similarly, the base length of the second single-stranded nucleic acid fragment 131 may be 10 to 200 bases. The length of the double-stranded nucleic acid 140 formed by hybridization of at least part of the first single-stranded nucleic acid fragment 121 with at least part of the second single-stranded nucleic acid fragment 131 may preferably be approximately 7 to 30 bases, or more preferably be 7 to 20 bases. The regions for hybridization in the first and second single-stranded nucleic acid fragments 121 and 131 may include the respective ends of these single-stranded nucleic acid fragments. The regions for hybridization in the first and second single-stranded nucleic acid fragments 121 and 131 do not have to include the ends of these single stranded nucleic acid fragments. For example, the regions may each be located at 7 to 13 bases from the end of the single stranded nucleic acid fragment. From the perspective of enhancing detection sensitivity, the regions for hybridization in the first and second single-stranded nucleic acid fragments 121 and 131 are preferred to include the respective ends of these single-stranded nucleic acid fragments.

### (Wells)

The method according to the present embodiment is suitable for detecting a target substance using digital measurements. When performing the method according to the present embodiment using a digital measurement, a well array in which multiple wells are arranged is preferred to be configured. The well array is preferred to be disposed in a channel of a fluidic device.

### (Fluidic device)

Fig. 2 is a schematic cross-sectional view illustrating an example of a fluidic device. As shown in Fig. 2, a fluidic device 200 includes a substrate 210 and a lid member 220 disposed facing the substrate 210. The lid member 220 includes a protrusion 221, and the end of the protrusion 221 is in contact with the substrate 210. The fluidic device 200 includes a well array 240 formed on one surface of the substrate 210, being integrated therewith. The surface of the substrate 210 provided with the well array 240 faces the lid member 220. The well array 240 includes a plurality of wells 241. The lid member 220 may be welded or bonded to the substrate 210.

The wells 241 are open to the surface of the substrate 210. The shape, size, and arrangement of the wells 241 are not specifically limited. However, it is preferred that one molecule of the target substance is introduced into each well 241. The wells 241 are preferred to be microwells with small volumes. For example, each well 241 may have a volume of approximately 10 fL to 100 pL. In the fluidic device 200, the wells 241 with the same shape and size configure the well array 240. The same shape and size may refer to the same shape and volume required for digital measurement, accepting a manufacturing error variation.

Each well 241 may have a diameter of, for example, approximately 1 µm to 10 µm. Each well 241 may have a depth of, for example, approximately 1 µm to 10 µm. The arrangement of the wells 241 is not specifically limited, and may be arranged, for example, in a triangular lattice shape or a square lattice shape, or may be randomly arranged.

In the fluidic device 200, there is a space formed between the well array 240 and the lid member 220 due to the presence of the projection 221. The space configures a channel 230. The channel 230 functions as a path for supplying a liquid in which a target substance, first specific binding substance, second specific binding substance, etc. are dispersed, and a sealing liquid described later. The shape, structure, volume, etc. of the channel 230 are not specifically limited, but the height of the channel 230 (i.e., the distance between the surface of a part of the substrate 210 facing the lid member 220 and including no well array, and the surface of the lid member 220 facing the substrate 210) is preferred to be, for example, 500 µm of less, more preferred to be, for example, 300 µm or less, even more preferred to be, for example, 200 µm or less, and most preferred to be, for example, 100 µm or less.

The protrusion 221 may be formed integrally with the lid member 220. For example, the lid member 220 can be formed by molding a thermoplastic resin fluid into a plate shape with a protrusion 221 using a mold. The lid member 220 may be provided with an inlet port 222 and an outlet port 223 for reagents.

If the lid member 220 has a protrusion 221, the lid member 220 and the substrate 210 are overlapped with each other so that the protrusion 221 is brought into contact with the surface of the substrate 210 in which the wells 241 are open. As a consequence, the space between the lid member 220 and the substrate 210 serves as a channel 230. The lid member 220 and the substrate 210 may be welded together by laser welding or the like.

### (Fluidic device modification 1)

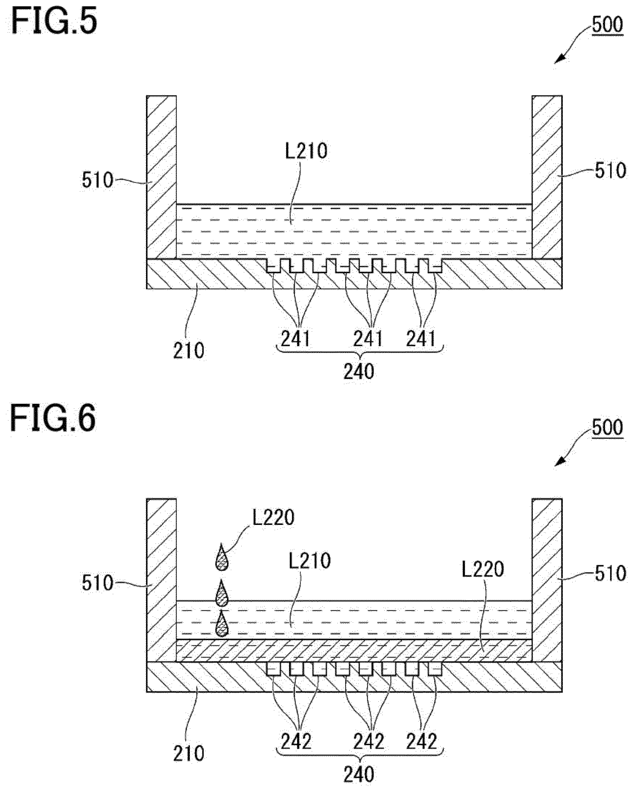

The fluidic device used for the method according to the present embodiment is not limited to the fluidic device 200 described above. Fig. 5 is a schematic cross-sectional view illustrating an example of a fluidic device. As shown in Fig. 5, a fluidic device 500 includes a substrate 210 and a wall member 510. The fluidic device 500 includes a well array 240 formed on one surface of the substrate 210, being integrated therewith. The well array 240 includes a plurality of wells 241.

The fluidic device 500 differs mainly from the fluidic device 200 described above in that it does not have a lid member 220. Accordingly, the fluidic device 500 has no channel.

### (Fluidic device modification 2)

In the fluidic device 200 described above, the lid member 220 is formed integrally with the protrusion 221. However, the lid member 220 and the protrusion 221 may be molded as separate bodies.

The fluidic devices 200 and 500 described above each include a well array 240 formed on one surface of the substrate 210, being integrated therewith. However, the well array does not have to be formed integrally with the substrate 210. For example, a well array 240 molded separately from the fluidic device may be disposed on the substrate 210 of the fluidic device. Alternatively, a resin layer may be deposited on the surface of the substrate 210, and a well array may be formed in the resin layer by etching or the like.

### (Materials of fluidic device)

The substrate 210 may be formed of a resin, for example. The type of the resin is not specifically limited, but it is preferred to be a resin resistant to the reagent and the sealing liquid. If the signal to be detected is fluorescence, the resin is preferred to have low autofluorescence. Examples of the resin include cycloolefin polymers, cycloolefin copolymers, silicones, polypropylenes, polycarbonates, polystyrenes, polyethylenes, polyvinyl acetates, fluororesins, and amorphous fluororesins, but are not limited thereto.

The substrate 210 may be provided with a plurality of wells 241 formed on one surface thereof in the thickness direction. Examples of the method of forming wells in a resin include injection molding, thermal imprinting, and optical imprinting.

Alternatively, for example, a fluororesin may be deposited on the substrate 210 and processed by etching or the like to form a well array. The fluororesin used may be, for example, CYTOP (trademark) (manufactured by AGC Inc.) or the like.

If the fluidic device includes a lid member 220, the material of the lid member 220 is preferred to be a resin with low autofluorescence, examples of which include thermoplastic resins such as cycloolefin polymers and cycloolefin copolymers.

The lid member 220 may be formed of a material that does not transmit light with a wavelength near the wavelength detected in fluorescence observation for signals, or a material that is completely opaque to light. For example, the lid member 220 may be formed of a thermoplastic resin with an addition of carbon, metal particles, etc.

### (Method according to the present embodiment)

Referring to Figs. 2 to 4 where necessary, the method according to the present embodiment will be described, taking an example of using the fluidic device 200. The method according to the present embodiment is a method of detecting a target substance in a sample, including a step of introducing into wells 241 a sample, a first specific binding substance 120 which is for a target substance 110 and labeled with a first single-stranded nucleic acid fragment 121, and a second specific binding substance 130 which is for the target substance 110 and labeled with a second single-stranded nucleic acid fragment 131, forming as a result a complex 100 containing the target substance 110, the first specific binding substance 120, and the second specific binding substance 130 if the target substance 110 is present in the sample, and forming a double-stranded nucleic acid 140 by hybridization of at least part of the first single-stranded nucleic acid fragment 121 with at least part of the second single-stranded nucleic acid fragment 131; and a step of detecting formation of the double-stranded nucleic acid 140, wherein detection of formation of the double-stranded nucleic acid 140 indicates presence of the target substance 110. According to the method of the present embodiment, a target substance can be detected without performing a washing process.

### <<Introduction process>>

First, as shown in Fig. 2, a reagent solution L210 is introduced from the inlet port 222 of the fluidic device 200 and supplied into the channel 230. The reagent solution L210 is a dispersion of a sample, a first specific binding substance 120, and a second specific binding substance 130, and contains a reagent for detecting formation of a double-stranded nucleic acid 140. If a target substance 110 is contained in the sample, the reagent solution L210 contains a first specific binding substance 120, a second specific binding substance 130, a target substance 110, and a reagent for detecting formation of a double-stranded nucleic acid 140.

The reagent solution L210 supplied into the channel 230 comes into contact with the well array 240. The reagent solution L210 is accommodated inside the wells 241. Consequently, the first specific binding substance 120, the second specific binding substance 130, the target substance 110, if present, and the reagent for detecting formation of a double-stranded nucleic acid 140 are introduced into the wells 241.

The reagent solution L210 can be prepared through the following first method. A sample, a first specific binding substance 120, and a second specific binding substance 130 are mixed together to undergo an antigen-antibody reaction between a target substance 110 that can be contained in the sample, and the first and second specific binding substances 120 and 130. The antigen-antibody reaction is performed, for example, at 25 to 37°C for 30 to 180 minutes. After that, a reagent for detecting formation of a double-stranded nucleic acid 140 is added to undergo an ICA reaction. The ICA reaction is allowed to undergo, for example, at 60 to 70°C for 10 to 60 minutes. The mixture after completion of the ICA reaction can be used as a reagent solution L210.

The reagent solution L210 can be prepared through the following second method. A sample, a first specific binding substance 120, and a second specific binding substance 130 are mixed together to prepare an antigen-antibody reaction solution. A reagent for detecting formation of a double-stranded nucleic acid 140 is added to the antigen-antibody reaction solution to prepare a mixture. The mixture is left to stand at 25 to 37°C for 30 to 180 minutes, for example, following which, the mixture is again left to stand at 60 to 70°C for 10 to 60 minutes, for example, to complete an ICA reaction. The mixture after completion of the ICA reaction can be used as a reagent solution L210.

The number of molecules of a target substance 110 introduced into each well 241 is not specifically limited, but 1 or less molecule, i.e., 0 or 1 molecule, of the target substance 110 is preferred to be introduced into each well 241. Thus, molecules of the target substance 110 can be detected on an individual basis, i.e., digital measurement can be performed. The target substance 110 is not required to be introduced into all the wells of the well array.

A means of introducing the target substance 110 into the wells is not specifically limited, but a means suitable for the selected target substance 110 can be selected. For example, the target substance 110 may be allowed to settle down in the fluidic device (in the channel) due to its own weight, and distributed to the wells. Alternatively, a substance capturing the target substance 110 (capture substance) may be used. Specifically, a target substance 110 unlikely to settle due to its own weight may be supplied with a capture substance bound thereto, or a capture substance may be immobilized in the wells in advance to capture the target substance 110 supplied, to improve the efficiency of introducing the target substance 110 into the wells.

A capture substance can be bound to the target substance 110 at any time during the method of the present embodiment. For example, before introducing the target substance 110 into the wells 241, binding may be performed by bringing the target substance 110 into contact with the capture substance in a sample tube. Alternatively, after introducing a capture substance into the wells 241, the target substance 110 may be introduced into the wells for contact with the capture substance in the wells.

The capture substance is a substance capable of capturing the target substance 110. The capture substance may be, for example, a conjugate of a solid phase and a specific binding substance for the target substance 110.

The solid phase may be particles, membranes, substrates, or the like. One type, or two or more types of the specific binding substance may be used for the target substance 110. For example, the number of types may be three, four, or five or more.

Particles are not specifically limited, but may be polymer particles, magnetic particles, glass particles, or the like. Particles are preferred to undergo surface treatment to avoid nonspecific adsorption. In order to immobilize the specific binding substance, it is preferred to use particles having a functional group such as a carboxyl group on the surface. More specifically, Magnosphere LC300 (product name) manufactured by JSR Corporation, or the like can be used.

Alternatively, for example, when a virus is used as the target substance 110, cells to which the virus can be attached (i.e., cells with virus receptors) may be used as a capture substance.

Examples of the first specific binding substance 120, the second specific binding substance 130, and the specific binding substance in the capture substance include antibodies, antibody fragments, and aptamers. Examples of the antibody fragments include Fab, F(ab')₂, Fab', single chain antibodies (scFvs), disulfide stabilized antibodies (dsFvs), dimeric V region fragments (diabodies), and peptides including CDRs. The antibodies may be monoclonal or polyclonal. The antibodies may be commercially available antibodies.

If the target substance 110 contains a sugar chain, the specific binding substance may be a lectin. If the target substance 110 contains a lipid membrane, the specific binding substance may be a substance bindable to lipid membranes. Examples of the substance bindable to lipid membranes include hydrocarbons such as hexanediol, and membrane proteins such as transmembrane proteins. Membrane proteins may include, for example, α-hemolysin, and the like.

The method of labelling a specific binding substance with a single-stranded nucleic acid fragment may include methods using cross-linking agents. The specific binding substance may be labelled with a single-stranded nucleic acid fragment via a linker molecule. Examples of the linker include polyethylene chains, hydrocarbon chains, and peptides, but are not specifically limited thereto. The single-stranded nucleic acid fragment may be DNA or RNA. The single-stranded nucleic acid fragment may contain an artificial nucleic acid such as BNA or LNA.

The method of immobilizing the specific binding substance on a particle surface is not specifically limited, but may be a method using physical adsorption, a method using chemical bonding, a method using avidin-biotin binding, a method using binding of protein G or protein A to an antibody, or the like. The method using physical adsorption may be a method of immobilizing the specific binding substance on a particle surface using hydrophobic interaction or electrostatic interaction. The method using chemical bonding may be a method using a crosslinking agent. For example, if the particle surface has a hydroxyl group, the carboxyl group of the specific binding substance may be reacted with a crosslinking agent to obtain an active ester, and then the hydroxyl group may be reacted with the ester group to immobilize the specific binding substance on the particle surface. It is preferred to provide a spacer between the specific binding substance and the particle surface so as not to inhibit the ability of the specific binding substance to recognize the target molecule.

As described above, when introducing the target substance 110 into the wells 241 using a capture substance, a conjugate of the capture substance and the target substance 110 is preferred to be formed under the condition that 0 or 1 molecule of the target substance 110 is captured by 1 molecule of the capture substance. The wells 241 are preferred to be configured so that 0 or 1 molecule of the capture substance is introduced into each well 241. Thus, digital measurement can be performed.

When the target substance 110, the first specific binding substance 120, and the second specific binding substance 130 are brought into contact with each other, complexes 100 each containing these substances are formed, with at least part of the first single-stranded nucleic acid fragment 121 hybridized with at least part of the second single-stranded nucleic acid fragment 131, forming a double-stranded nucleic acid 140. The complexes 100 may be formed in a sample tube before introduction of the sample and the first and second specific binding substances 120 and 130 into the wells, or may be formed in the wells 241.

The target substance may be formed of two-molecule units, and may be detected using a nucleic acid-labeled specific binding substance specific to the respective molecules. In this case, the two molecules can be measured as to whether they are in a bound state. If the two molecules form a homodimer, the first nucleic acid-labeled specific binding substance and the second nucleic acid-labeled specific binding substance may recognize the same epitope.

### <<Sealing process>>

After introducing a sample, a first specific binding substance 120, and a second specific binding substance 130 into the wells 241, the openings of the wells 241 may be sealed.

The method of sealing the openings of the wells 241 is not specifically limited as long as the liquid accommodated in each well 241 is not mixed with the liquid accommodated in another well 241. For example, the openings of the wells 241 may be sealed by covering them with a sealing liquid. Alternatively, the openings of the wells 241 may be sealed by laminating a platelike member such as a glass plate on them.

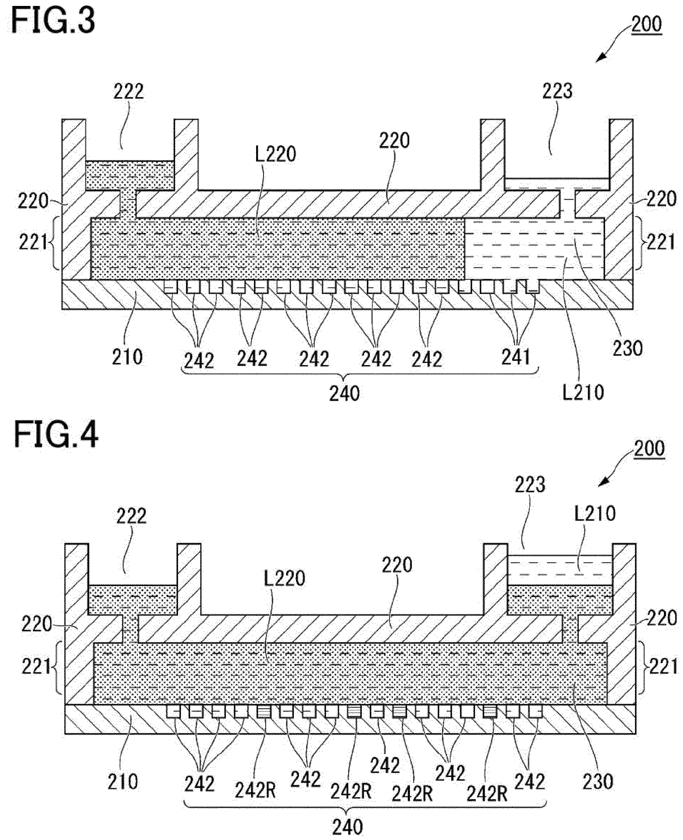

For example, as shown in Fig. 3, a sealing liquid L220 may be supplied into the channel 230 between the substrate 210 and the lid member 220 from the inlet port 222 of the lid member 220. The sealing liquid L220 supplied into the channel 230 may come into contact with the well array 240. The sealing liquid L220 may expel and replace the reagent solution L210 supplied into the channel 230 and is not accommodated in the wells 241. Thus, the sealing liquid L220 may seal the plurality of wells 241 in which the reagent solution L210 containing the target substance 110 is accommodated, and the wells 241 may become independent reaction spaces (microcompartments 242). When the channel 230 is filled with the sealing liquid L220, excess sealing liquid L220 may be discharged from the outlet port 223. Fig. 4 shows a state in which all the wells 241 of the well array 240 are sealed with the sealing liquid L220, forming sealed wells (i.e., microcompartments) 242.

Alternatively, a lipid may be dissolved in the reagent solution L210 and, after supplying the sealing liquid L220 into the channel 230, the solution containing the lipid may be additionally supplied to form a lipid bilayer membrane at the openings of the wells 241, so that the plurality of wells 241 are individually sealed with the lipid bilayer membrane, forming sealed wells 242. Examples of the lipid to form the lipid bilayer membrane include 1,2-dioleoyl-sn-glycero-3-phosphoethanolamine (DOPE), 1,2-dioleoyl-sn-glycero-3-phosphoglycerol (DOPG), and mixtures thereof, but are not limited thereto.

The wells 242 sealed using any of the methods described above may include molecules of the first specific binging substance 120 not bound to the target substance 110 and/or molecules of the second specific binging substance 130 not bound to the target substance 110.

The sealing liquid can seal the individual liquids introduced into the plurality of wells 241 to form droplets (which are also referred to as microdroplets) to prevent mixture of the liquids. The sealing liquid is preferred to be an oil-based solution, and more preferred to be an oil. Examples of the oil that can be used include fluorine oils, silicone oils, hydrocarbon oils, and mixtures thereof. More specifically, FC-40 (product name) manufactured by Sigma-Aldrich, or the like can be used. FC-40 (CAS Number: 86508-42-1) is a fluorinated aliphatic compound with a specific gravity of 1.85 g/mL at 25°C.

### <<Detection process>>

Subsequently, formation of a double-stranded nucleic acid 140 is detected. The double-stranded nucleic acid 140 is preferred to be detected using a signal amplification reaction. For example, the signal amplification reaction may be an invasive cleavage assay (ICA).

The ICA reaction relates to the principle that signal amplification progresses through two reaction cycles which are (1) complementary binding between nucleic acids, and (2) recognition and cleavage of a triple-stranded structure by enzymes.

The ICA reaction is less affected by reaction cycle inhibition due to foreign substances. Accordingly, use of the ICA reaction can contribute to detecting the target substance 110 with high accuracy. If the ICA reaction is used as the signal amplification reaction, the reagent solution L210 (solution containing a target substance 110, a first specific binding substance 120, and a second specific binding substance 130) preferably contains a reaction reagent required for the ICA reaction.

Examples of the reaction reagent required for the ICA reaction include ICA reaction reagents such as flap probes, flap endonucleases (FENs), and fluorescent substrates. Flap probes are nucleic acid fragments designed to hybridize with the first single-stranded nucleic acid fragment 121 or the second single-stranded nucleic acid fragment 131 to form a flap structure with the double-stranded nucleic acid 140.

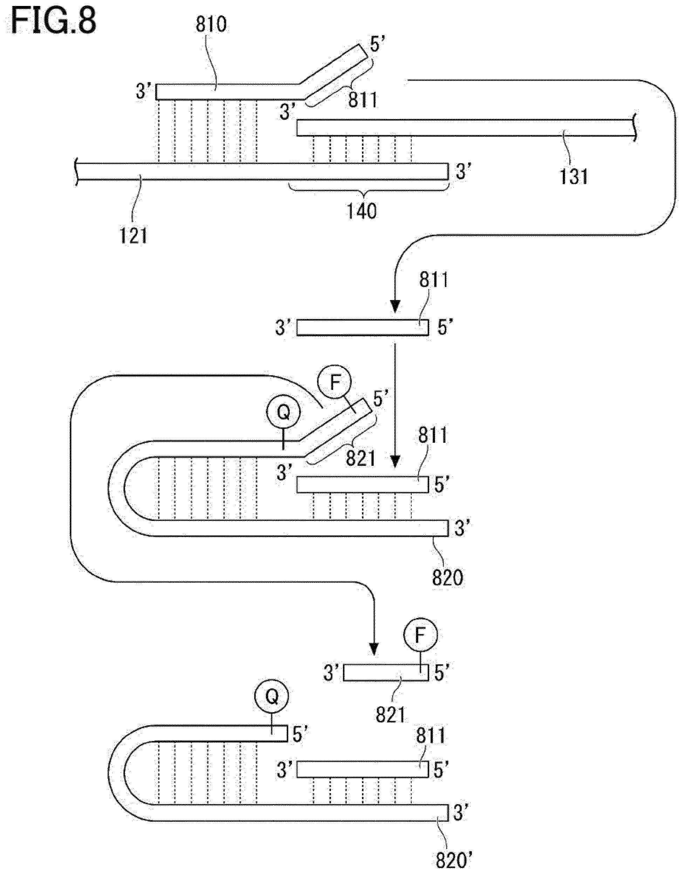

Fig. 8 is a schematic diagram illustrating an example of an ICA method. Fig. 8 shows an example of detecting a double-stranded nucleic acid 140 formed by hybridization of at least part of the first single-stranded nucleic acid fragment 121 with at least part of the second single-stranded nucleic acid fragment 131 using an ICA method.

First, a flap probe is hybridized with the first single-stranded nucleic acid fragment 121 or the second single-stranded nucleic acid fragment 131. In the example shown in Fig. 8, a flap probe 810 is hybridized with the first single-stranded nucleic acid fragment 121. Consequently, a first flap site 811 is formed.

Subsequently, an FEN is reacted with the first flap site 811, by which the first flap site 811 is cleaved to produce a nucleic acid fragment 811. Subsequently, the nucleic acid fragment 811 is hybridized with a fluorescent substrate (nucleic acid fragment 820) to form a second flap site 821.

In the example shown in Fig. 8, a fluorescent substance F is bound to the 5' end of the nucleic acid fragment 820, and a quenching substance Q is bound to the 3' side a few bases from the 5' end of the nucleic acid fragment 820. Subsequently, the second flap site 821 is reacted with an FEN, by which the second flap site 821 is cleaved to produce a nucleic acid fragment 821. Consequently, the fluorescent substance F is separated from the quenching substance Q and generates a fluorescent signal. By detecting the fluorescent signal, formation of a double-stranded nucleic acid 140 can be detected.

The reagent solution L210 can be a liquid that is generally used in biochemical analyses performed using fluidic devices, and is preferred to be an aqueous solution. A surfactant or the like may be added to the reagent solution L210 to facilitate sealing of liquid in the wells.

When using the ICA reaction to detect formation of a double-stranded nucleic acid 140, if a double-stranded nucleic acid 140 is present, the fluorescent substance F is released from the quenching substance Q due to an isothermal enzymatic reaction, and a predetermined fluorescent signal is emitted in response to the excitation light.

For detecting formation of a double-stranded nucleic acid 140, a known suitable method can be selected according to the type of the signals to be detected. For example, when observing fluorescent signals, excitation light corresponding to the fluorescent substance may be applied to the microcompartments 242 to observe fluorescence emitted by the fluorescent substance. For example, as shown in Fig. 4, a predetermined reaction may be allowed to occur in the microcompartments 242, and generated signals may be observed. Microcompartments 242R are wells where signals have been detected, and microcompartments 242 are wells where no signals have been detected.

Referring to Figs. 5 to 7, a method according to the present embodiment will be described, taking an example of using the fluidic device 500.

First, as shown in Fig. 5, a reagent solution L210 is introduced into the fluidic device 500. The reagent solution L210 is a dispersion of a target substance 110, a first specific binding substance 120, and a second specific binding substance 130, and contains a reagent for detecting formation of a double-stranded nucleic acid 140. In the reagent solution L210, the concentration of the target substance 110 is preferred to be adjusted such that 1 molecule or less of the target substance is accommodated in each well 241.

Subsequently, as shown in Fig. 6, a sealing liquid L220 is introduced into the fluidic device 500. The specific gravity of the sealing liquid L220 is larger than that of the reagent solution L210. Therefore, the sealing liquid L220 sinks below the reagent solution L210 not accommodated in the wells 241 and contacts the well array 240. Thus, the sealing liquid L220 seals the plurality of wells 241 in which the reagent solution L210 containing the target substance is accommodated, and the wells 241 become independent reaction spaces (i.e., microcompartments 242).

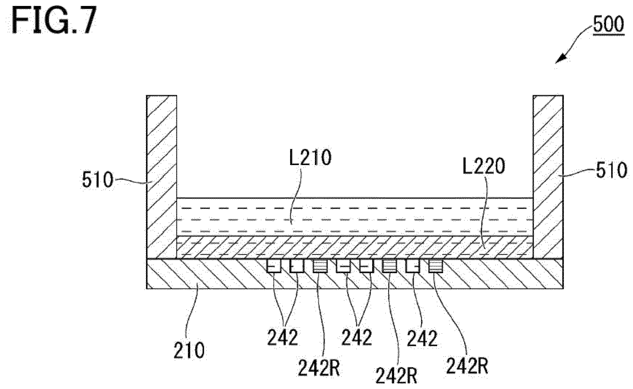

Subsequently, as shown in Fig. 7, a predetermined reaction is allowed to occur in the microcompartments 242, and generated signals are observed. Microcompartments 242R are wells where signals have been detected, and microcompartments 242 are wells where no signals have been detected.

### [Complex]

In an embodiment, the present invention provides a complex 100 containing a target substance 110, a first specific binding substance 120 which is for the target substance 110 and labeled with a first single-stranded nucleic acid fragment 121, and a second specific binding substance 130 which is for the target substance 110 and labeled with a second single-stranded nucleic acid fragment 131, and forming a double-stranded nucleic acid 140 by hybridization of at least part of the first single-stranded nucleic acid fragment 121 with at least part of the second single-stranded nucleic acid fragment 131.

In the complex 100, a flap probe may be further hybridized with the first single-stranded nucleic acid fragment 121 or the second single-stranded nucleic acid fragment 131.

As described above, a target substance can be detected without performing a washing process, by using the complex according to the present embodiment.

### [Kit]

In an embodiment, the present invention provides a kit for detecting a target substance 110, including a well array 240 having a plurality of wells 241, a first specific binding substance 120 which is for the target substance 110 and labeled with a first single-stranded nucleic acid fragment 121, and a second specific binding substance 130 which is for the target substance 110 and labeled with a second single-stranded nucleic acid fragment 131.

The method of detecting a target substance described above can be preferably performed using the kit according to the present embodiment. Accordingly, the kit according to the present embodiment can be said to be a kit which is used for the method including a step of introducing into wells 241 a target substance 110, a first specific binding substance 120 which is for the target substance 110 and labeled with a first single-stranded nucleic acid fragment 121, and a second specific binding substance 130 which is for the target substance 110 and labeled with a second single-stranded nucleic acid fragment 131, forming as a result a complex 100 containing the target substance 110, the first specific binding substance 120, and the second specific binding substance 130, and forming a double-stranded nucleic acid 140 by hybridization of at least part of the first single-stranded nucleic acid fragment 121 with at least part of the second single-stranded nucleic acid fragment 131; and a step of detecting formation of the double-stranded nucleic acid 140, wherein detection of formation of the double-stranded nucleic acid 140 indicates presence of the target substance 110.

The well array may be disposed inside the fluidic device described above. In the kit according to the present embodiment, the target substance, the first single-stranded nucleic acid fragment, the first specific binding substance, the second single-stranded nucleic acid fragment, and the second specific binding substance are similar to those which are described above.

The kit according to the present embodiment may further include a sealing liquid L220 for sealing the openings of the wells 241. The sealing liquid L220 is similar to the sealing liquid described above.

The kit according to the present embodiment may further include a reagent for detecting a double-stranded nucleic acid 140 formed by hybridization of at least part of the first single-stranded nucleic acid fragment 121 with at least part of the second single-stranded nucleic acid fragment 131.

Examples of such a reagent include ICA reaction reagents, specifically including flap probes, flap endonucleases (FENs), and fluorescent substrates.

### Examples

### [Materials and methods]

### (Detection of prostate-specific antigen (PSA))

### <<Oligonucleotide modification to antibody 1>>

Using an antibody-oligonucleotide conjugation tool, two types of anti-human PSA mouse monoclonal antibodies (clones 5A6 and 1H12 manufactured by HyTest Ltd.) were conjugated to different oligonucleotides (manufactured by FASMAC). Oligonucleotides with amino group modification at the 5' ends, i.e., DNA 1 (5'-TTTGTCACTGTCTCTCTCTTTTCTTTCTGTGAGCAATTTCACCCAA-3', sequence number 1) and DNA 2 (5'-GCATGGTTCCAATTTGGGTGAT-3', sequence number 2), were conjugated. The length of the double-stranded nucleic acid formed by hybridization of the DNA 1 and DNA 2 is 9 bases.

### <<Antigen-antibody reaction 1>>

A target substance (PSA), the above two types of oligonucleotide-modified antibodies, and a blocking buffer were mixed with each other to prepare mixtures. The volume of each mixture was 10 µL. Target substances were prepared to have a final concentration of 0 nM, 0.294 nM, 2.94 nM, 14.7 nM, and 29.4 nM. The two types of oligonucleotide-modified antibodies were each prepared to have a final concentration of 8.56 nM. As the blocking buffer, tris buffered saline (TBS) containing 1% bovine serum albumin (BSA) was used. Subsequently, each mixture was allowed to undergo a reaction at 37°C for 30 minutes.

### <<Preparation of invasive cleavage assay (ICA) reaction reagent 1>>

An ICA reaction reagent for detection was prepared to perform an ICA reaction using the above oligonucleotides modified to the antibodies. The ICA reaction reagent in the present experimental example contained 0.25 µM allele probe (5'-CGCGCCGAGGAATTGCTCACAGAAAGGA-3') (manufactured by FASMAC, sequence number 3), 2 µM FRET cassette 1 (fluorescent substrate, Alexa 488-BHQ: X-TTCT-Y-AGCCGGTTTTCCGGCTGAGACCTCGGCGCG, X: Alexa 488 + Amino C6, Y: Black hole quencher (BHQ) 1-dT) (manufactured by Japan Bio Services, sequence number 4), 0.81 µM flap endonuclease (FEN)-1, 50 mM Tris-HCl (pH 8.5), 20 mM MgCl₂, and 0 .05% Tween 20. The concentrations of the components in the ICA reaction reagent are the final concentrations in the experimental example.

### [Experimental Example 1]

### (Detection of target substance (PSA) inside tube)

10 µL of each mixture after the antigen-antibody reaction was mixed with 10 µL of the above ICA reaction reagent to undergo a reaction at 66°C for 1 hour using Rotor-Gene Q (manufactured by QIAGEN) to detect Alexa 488 fluorescence. This reaction may hereinafter be referred to as an immuno-ICA reaction.

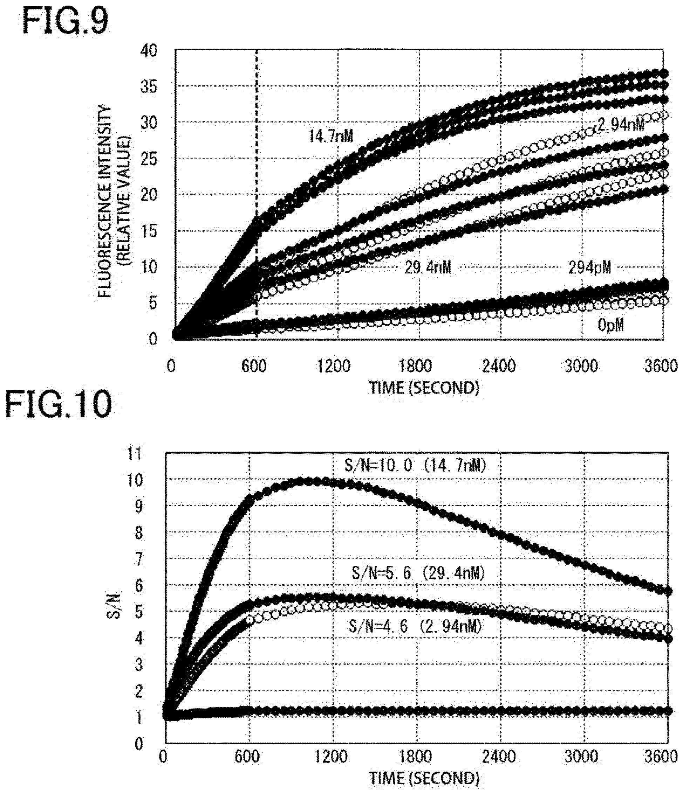

Figs. 9 to 11 are graphs showing the results of Experimental Example 1. Fig. 9 shows the results of immuno-ICA reactions using the PSA antigen of 0 nM, 0.294 nM, 2.94 nM, 14.7 nM, or 29.4 nM. In Fig. 9, the vertical axis indicates Alexa 488 fluorescence intensity (relative value), and the horizontal axis indicates time (second) after start of ICA reaction. It became clear that the presence or absence of the PSA antigen could be detected under the conditions of 2.94 nM or higher.

Fig. 10 is a graph showing the results of calculating signal-to-noise ratios (S/N ratios) in the results shown in Fig. 9, with the fluorescence intensity at the antigen concentration of 0 nM being N (noise), and the fluorescence intensity at other concentrations being S (signal). In Fig. 10, the vertical axis indicates S/N ratio, and the horizontal axis indicates time (second) after start of ICA reaction. The S/N ratio under the condition of the antigen concentration being 14.7 nM was 10.0 at 1,080 seconds from the start of the reaction. The S/N ratio under the condition of the antigen concentration being 29.4 nM was 5.6 at 1,080 seconds from the start of the reaction. The S/N ratio under the condition of the antigen concentration being 2.94 nM was 4.6 at 1,380 seconds from the start of the reaction.

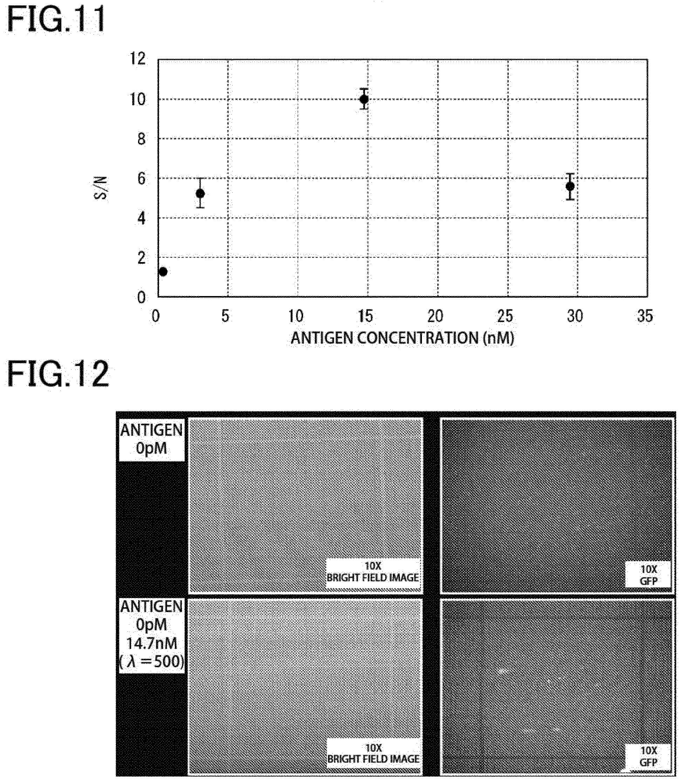

Fig. 11 is a graph showing the relationship between antigen concentration (nM) and S/N ratio 18 minutes after start of the reaction. In Fig. 11, the vertical axis indicates S/N ratio, and the horizontal axis indicates antigen concentration (nM). The antigen concentration of 14.7 nM showed the highest S/N ratio of around 10.

### [Experimental Example 2]

### (Detection of target substance (PSA) using fluidic device)

10 µL of each mixture after the antigen-antibody reaction was mixed with 10 µL of the above ICA reaction reagent, and 10 µL of the resultant mixture was supplied into a fluidic device. Then, FC-40 (manufactured by Sigma) was supplied as a sealing liquid to seal the wells. The fluidic device was set on a hot plate to undergo a reaction at 66°C for 20 minutes.

Subsequently, fluorescence images of the wells in the fluidic device were captured using a fluorescence microscope BZ-710 (manufactured by KEYENCE Corporation) with a 10× objective lens. The exposure time for each image was 2 seconds using a GFP fluorescence filter. After sealing the wells, bright field images were also captured using a microscope BZ-710 with a 10x objective lens.

The upper-left image in Fig. 12 is a bright field image at the antigen concentration of 0 pM. The upper-right image in Fig. 12 is a fluorescence image at the antigen concentration of 0 pM. The lower-left image in Fig. 12 is a bright field image at the antigen concentration of 14.7 pM. The lower-right image in Fig. 12 is a fluorescence image at the antigen concentration of 14.7 pM. As a consequence, fluorescence signals derived from the target substance (PSA) could be observed in the wells where the antigen was present.

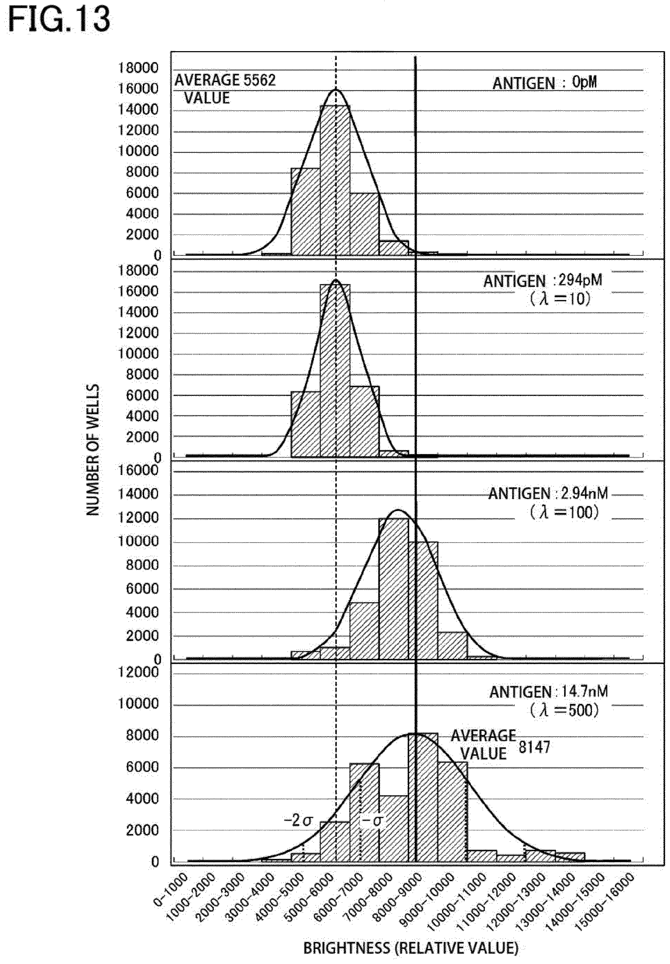

Fig. 13 show graphs indicating the number of wells and brightness thereof where fluorescence was observed, in the case where the target substance with each concentration was detected. In Fig. 13, the vertical axis indicates the number of wells where fluorescence was observed, and the horizontal axis indicates brightness (relative value). It became clear that the number of wells and brightness thereof where fluorescence was observed increased, relying on the increase in the antigen concentration.

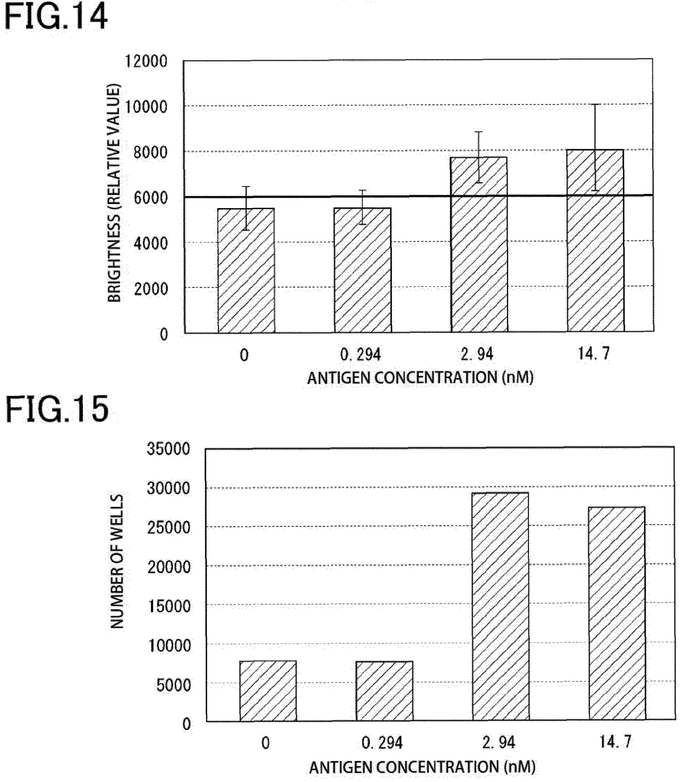

Fig. 14 is a graph showing the average value ± standard deviation of brightness in the case where the target substance with each concentration was detected. In Fig. 14, the vertical axis indicates brightness (relative value), and the horizontal axis indicates antigen concentration. Fig. 15 is a graph showing the number of wells with a brightness higher than a tentatively set threshold brightness (6,000 in the present experimental example) in the case where the target substance with each concentration was detected. It became clear that approximately 3.7 times the number of wells showed brightness equal to or higher than the threshold when the antigen concentration was 2.94 nM or higher, compared to the case where there was no antigen.

### <<Oligonucleotide modification to antibody 2>>

Using an antibody-oligonucleotide conjugation tool, two types of anti-human PSA mouse monoclonal antibodies (clones 5A6 and 1H12 manufactured by HyTest Ltd.) were conjugated to different oligonucleotides (manufactured by FASMAC). Oligonucleotides with amino group modification at the 5' ends, i.e., DNA 3 (5'-TTTGTCACTGTTCCTCCTTTTGTTTTCCTTTCTGTGAGCAATTTC-3', sequence number 5), DNA 4 (5'-TTTGTCACTGTTCCTCCTTTTGTTTTCCTTTCTGTGAGCAATTTCACCCAAATT-3', sequence number 6), and DNA 5 (5'-TTTGTCACTGTTCCTCCTTTTGTTTTCCTTTCTGTGAGCAATTTCACCCAAATTGGA-3', sequence Number 7) were conjugated. The length of the double-stranded nucleic acid formed by hybridization of DNA 2 and DNA 3 is 3 bases. The length of the double-stranded nucleic acid formed by hybridization of DNA 2 and DNA 4 is 12 bases. The length of the double-stranded nucleic acid formed by hybridization of DNA 2 and DNA 5 is 15 bases.

### <<Antigen-antibody reaction 2>>

A target substance (PSA), the oligonucleotide-modified antibodies conjugated to DNA 1 and DNA 2, and a blocking buffer were mixed with each other to prepare mixtures. The volume of each mixture was 10 µL. The target substance was prepared to have a final concentration of 0 nM or 2.94 nM. The two types of oligonucleotide-modified antibodies were each prepared to have a final concentration of 8.56 nM. As the blocking buffer, TBS containing 1% BSA was used. Subsequently, each mixture was allowed to undergo a reaction at 37°C for 30 minutes. Each mixture was prepared using the same method, except that a combination of oligonucleotide-modified antibodies conjugated to DNA 2 and DNA 3, a combination of oligonucleotide-modified antibodies conjugated to DNA 2 and DNA 4, or a combination of oligonucleotide-modified antibodies conjugated to DNA 2 and DNA 5 was used as the two types of oligonucleotide-modified antibodies instead of the combination mentioned above, and the mixture was allowed to undergo a reaction under the same conditions.

### <<Preparation of ICA reaction reagent 2>>

An ICA reaction reagent for detection was prepared to perform an ICA reaction using the above oligonucleotides modified to the antibodies. The ICA reaction reagent in the present experimental example contained 0.25 µM allele probe (5'-CGCGCCGAGGAATTGCTCACAGAAAGGA-3') (manufactured by FASMAC, sequence number 3), 4 µM FRET cassette 1 (fluorescent substrate, Alexa 488-BHQ: X-TTCT-Y-AGCCGGTTTTCCGGCTGAGACCTCGGCGCG, X: Alexa 488 + Amino C6, Y: Black hole quencher (BHQ) 1-dT) (manufactured by Japan Bio Services, sequence number 4), 0.81 µM flap endonuclease (FEN)-1, 50 mM Tris-HCl (pH 8.5), 20 mM MgCl₂, and 0 .05% Tween 20. The concentrations of the components in the ICA reaction reagent are the final concentrations in the experimental example.

### [Experimental Example 3]

### (Effect of double-stranded nucleic acid length formed by hybridization)

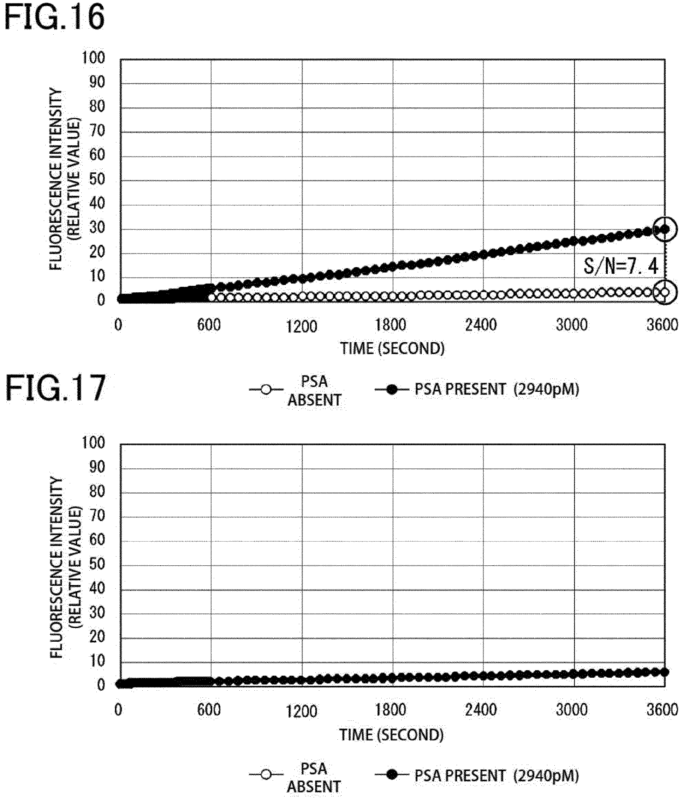

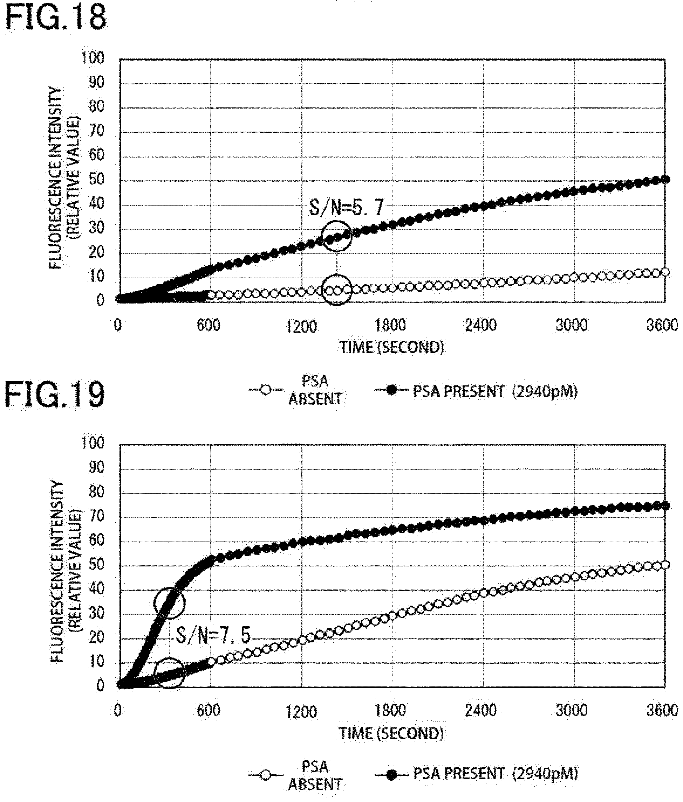

10 µL of each mixture after the antigen-antibody reaction was mixed with 10 µL of the ICA reaction reagent prepared through the method described in <<Preparation of ICA reaction reagent 2>> to undergo a reaction at 66°C for 1 hour using Rotor-Gene Q (manufactured by QIAGEN) to detect Alexa 488 fluorescence. Fig. 16 is a graph showing the results of the immuno-ICA reaction using oligonucleotide-modified antibodies conjugated to DNA 1 and DNA 2. Fig. 17 is a graph showing the results of the immuno-ICA reaction using oligonucleotide-modified antibodies conjugated to DNA 2 and DNA 3. Fig. 18 is a graph showing the results of the immuno-ICA reaction using oligonucleotide-modified antibodies conjugated to DNA 2 and DNA 4. Fig. 19 is a graph showing the results of the immuno-ICA reaction using oligonucleotide-modified antibodies conjugated to DNA 2 and DNA 5.

From the results shown in Figs. 16 to 19, it was found that the longer the double-stranded nucleic acid formed by hybridization, the better the reactivity. On the other hand, the longer the double-stranded nucleic acid formed by hybridization, the higher the background reaction signals. However, when the double-stranded nucleic acid formed by hybridization was 9 bp (Fig. 16), the S/N ratio 3,600 seconds after start of the reaction was 7.4, when the double-stranded nucleic acid formed by hybridization was 12 bp (Fig. 18), the S/N ratio 1,440 seconds after start of the reaction was 5.7, and when the double-stranded nucleic acid formed by hybridization was 15 bp (Fig. 19), the S/N ratio 288 seconds after start of the reaction was 7.5. Accordingly, it was found that, even when the double-stranded nucleic acid formed by hybridization was 12 to 15 bp and the background reaction signals were relatively high, the target substance could be detected with sufficiently high sensitivity.

### [Experimental Example 4]

### (Effect of mixing antigen-antibody reaction solution and ICA reaction solution)

10 µL of each mixture after the antigen-antibody reaction was mixed with 10 µL of the ICA reaction reagent to undergo an immuno-ICA reaction as in Experimental Example 3, except that the final concentration of the target substance (PSA) was 0 nM or 2.94 nM, and the final concentration of the oligonucleotide-modified antibodies conjugated to DNA 2 and DNA 5 was 8.56 nM in the mixture (hereinafter referred to as Reaction Type I).

The target substance (PSA), the oligonucleotide-modified antibodies conjugated to DNA 2 and DNA 5, and a blocking buffer were mixed with each other to prepare mixtures. The volume of each mixture was 10 µL. The target substance in each mixture was prepared to have a final concentration of 0 nM or 2.94 nM. The two types of oligonucleotide-modified antibodies were each prepared to have a final concentration of 8.56 nM. 10 µL of each mixture was mixed with 10 µL of the ICA reaction reagent prepared through the method described in <<Preparation of ICA reaction reagent 2>>. In the obtained mixture, the final concentration of the target substance was 0 nM or 1.47 nM, and the final concentration of the two types of oligonucleotide-modified antibodies was 4.28 nM. This mixture was left to stand at 37°C for 30 minutes to undergo an antigen-antibody reaction, and then left to stand at 66°C for 1 hour to undergo an immuno-ICA reaction to detect Alexa 488 fluorescence using Rotor-Gene Q (manufactured by QIAGEN) (hereinafter referred to as Reaction Type II).

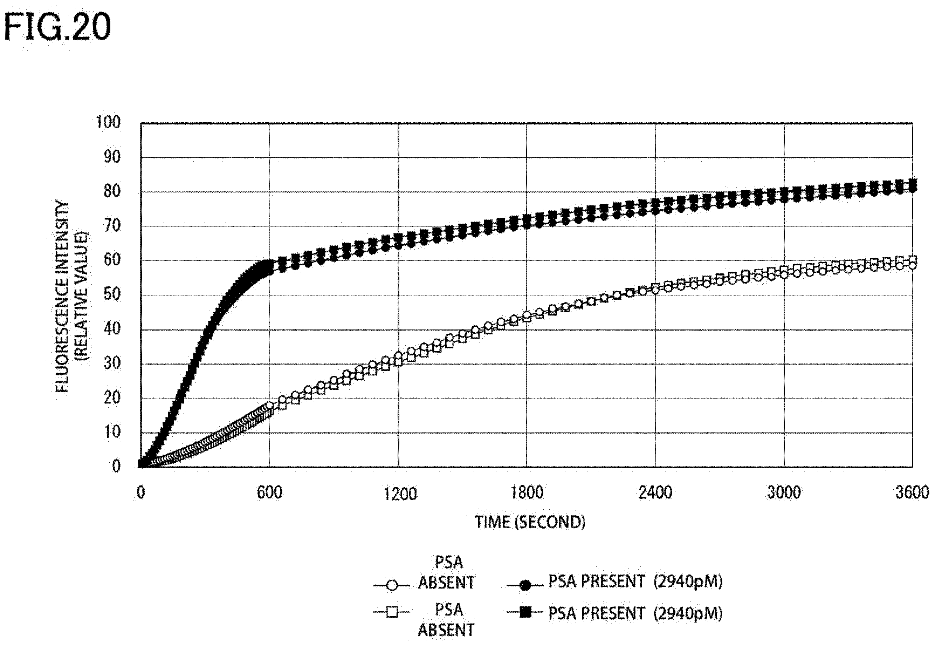

Fig. 20 shows the results of Reaction Types I and II. Compared to the method of Reaction Type I in which the ICA reaction solution is mixed after the antigen-antibody reaction, Reaction Type II showed no significant difference in ICA reaction intensity and background reaction intensity. From this result, it was found that the ICA reaction reagent did not weaken the antigen-antibody reaction, and the antigen-antibody reaction reagent did not weaken the ICA reaction. It is an unanticipated effect that the immuno-ICA reaction can be detected with high sensitivity, although the process is simpler in that the antigen-antibody reaction and immuno-ICA reaction can be sequentially performed after mixing the antigen-antibody reaction solution with the ICA reaction reagent.

### [Industrial Applicability]

According to the present invention, a technique for detecting a target substance without performing a washing process can be provided.

### [Reference Signs List]

- 100: Complex
- 110: Target substance
- 111: First binding site
- 112: Second binding site
- 120: First specific binding substance
- 121: First single-stranded nucleic acid fragment
- 130: Second specific binding substance
- 131: Second single-stranded nucleic acid fragment
- 140: Double stranded nucleic acid
- 200, 500: Fluidic device
- 210: Substrate
- 220: Lid member
- 221: Projection
- 222: Inlet port
- 223.: Outlet port
- 230: Channel
- 240: Well array
- 241: Well
- 242 ....: Sealed well (microcompartment)
- L210: Reagent solution
- L220: Sealing liquid
- 242R: Signal-detected well
- 510: Wall member
- 810: Flap probe
- 811: First flap site (nucleic acid fragment)
- 821: Second flap site (nucleic acid fragment)
- 820, 820': Nucleic acid fragment
- F: Fluorescent substance
- Q: Quenching substance

## Claims

1. a method of detecting a target substance in a sample, comprising
a step of introducing into wells the sample, a first specific binding substance which is for the target substance and labeled with a first single-stranded nucleic acid fragment, and a second specific binding substance which is for the target substance and labeled with a second single-stranded nucleic acid fragment, forming as a result a complex containing the target substance, the first specific binding substance, and the second specific binding substance if the target substance is present in the sample, and forming a double-stranded nucleic acid by hybridization of at least part of the first single-stranded nucleic acid fragment with at least part of the second single-stranded nucleic acid fragment; and
a step of detecting formation of the double-stranded nucleic acid, wherein
detection of formation of the double-stranded nucleic acid indicates presence of the target substance.

2. The method according to claim 1, wherein the wells each have a volume of 10 fL to 100 pL.

3. The method according to claim 1 or 2, wherein the first specific binding substance recognizes a first binding site of the target substance and the second specific binding substance recognizes a second binding site of the target substance, and the first binding site and the second binding site are different from each other.

4. The method according to claim 3, wherein a distance between the first binding site and the second binding site is a distance at which at least part of the first single-stranded nucleic acid fragment can be hybridized with at least part of the second single-stranded nucleic acid fragment.

5. The method according to claim 1 or 2, wherein the first single-stranded nucleic acid fragment and the second single-stranded nucleic acid fragment each have a base length of 10 to 200 bases.

6. The method according to claim 1 or 2, further comprising a step of sealing openings of the wells after introducing the sample, the first specific binding substance, and the second specific binding substance into the wells.

7. The method according to claim 6, wherein the wells whose openings are sealed include molecules of the first specific binding substance unbound to the target substance and/or molecules of the second specific binding substance unbound to the target substance.

8. The method according to claim 1 or 2, wherein the target substance is a substance selected from a group consisting of proteins, sugar chains, nucleic acids, lipid membrane structures, bacteria, viruses, and cells, or a composite substance thereof.

9. The method according to claim 1 or 2, wherein the first specific binding substance and the second specific binding substance each are a substance selected from a group consisting of antibodies, lectins, and substances bindable to lipid membranes.

10. The method according to claim 1 or 2, wherein the step of detecting formation of the double-stranded nucleic acid is performed through an invasive cleavage assay.

11. A complex containing a target substance, a first specific binding substance which is for the target substance and labeled with a first single-stranded nucleic acid fragment, and a second specific binding substance which is for the target substance and labeled with a second single-stranded nucleic acid fragment, and forming a double-stranded nucleic acid by hybridization of at least part of the first single-stranded nucleic acid fragment with at least part of the second single-stranded nucleic acid fragment.

12. A kit for detecting a target substance, comprising
a well array including a plurality of wells;
a first specific binding substance which is for the target substance and labeled with a first single-stranded nucleic acid fragment;
and a second specific binding substance which is for the target substance and labeled with a second single-stranded nucleic acid fragment.

13. The kit according to claim 12, further comprising a sealing liquid for sealing the openings of the wells.

14. The kit according to claim 12 or 13, further comprising a reagent for detecting a double-stranded nucleic acid formed by hybridization of at least part of the first single-stranded nucleic acid fragment with at least part of the second single-stranded nucleic acid fragment.
